# EUROPEAN PATENT SPECIFICATION

(11) **EP 3 559 276 B1**
(45) Date of publication and mention of the grant of the patent: **23.03.2022**
(21) Application number: 17817756.4
(22) Date of filing: 20.12.2017
(51) Int. Cl.: C12Q 1/6886

(54) **IDENTIFICATION OF DRUGS TARGETING NON-GENETIC DRUG TOLERANCE PROGRAMS IN CANCER**
IDENTIFIZIERUNG VON AUF NICHT GENETISCHE ARZNEIMITTELTOLERANZPROGRAMME GERICHTETEN MEDIKAMENTEN BEI KREBS
IDENTIFICATION DE MÉDICAMENTS CIBLANT DES PROGRAMMES DE TOLÉRANCE DE MÉDICAMENT NON-GÉNÉTIQUES DANS LE CANCER

(30) Priority: 20.12.2016 EP 16205346
(43) Date of publication of application: 30.10.2019
(73) Proprietor: ETH Zurich, 8092 Zurich (CH)
(72) Inventor: KREK, Wilhelm, 8704 Herrliberg (CH); FLÜCKIGER-MANGUAL, Stefanie, 8050 Zürich (CH)
(74) Representative: Ledl, Andreas
(86) International application number: PCT/EP2017/083868
(87) International publication number: WO 2018/115150

(56) References cited:
- WO-A1-2011/112678
- WO-A2-2014/147573
- S MICHAEL ROTHENBERG ET AL: "Inhibition of mutant EGFR in lung cancer cells triggers SOX2-FOXO6-dependent survival pathways", ELIFE, vol. 4, 16 February 2015 (2015-02-16), XP055380907, DOI: 10.7554/eLife.06132
- YAJUN TIAN ET AL: "SOX2 oncogenes amplified and operate to activate AKT signaling in gastric cancer and predict immunotherapy responsiveness", JOURNAL OF CANCER RESEARCH AND CLINICAL ONCOLOGY., vol. 140, no. 7, 22 April 2014 (2014-04-22), pages 1117-1124, XP055380375, DE ISSN: 0171-5216, DOI: 10.1007/s00432-014-1660-0
- D. E. LINN ET AL: "A Role for OCT4 in Tumor Initiation of Drug-Resistant Prostate Cancer Cells", GENES & CANCER, vol. 1, no. 9, 1 September 2010 (2010-09-01), pages 908-916, XP055169326, ISSN: 1947-6019, DOI: 10.1177/1947601910388271
- FANG XUEFENG ET AL: "The SOX2 response program in glioblastoma multiforme: an integrated ChIP-seq, expression microarray, and microRNA analysis", BMC GENOMICS, BIOMED CENTRAL LTD, LONDON, UK, vol. 12, no. 1, 6 January 2011 (2011-01-06), page 11, XP021086400, ISSN: 1471-2164, DOI: 10.1186/1471-2164-12-11

## Description

The present invention relates to a cell-based modular screening approach to identify compounds useful in the therapy of cancer, in particular, but not exclusively, in combination with inhibitors of the MAPK and EGFR pathway.

### Background

Genetic alterations of cancer cells often affect genes that are important for cell cycle control, proliferation, differentiation and/or signal transduction (Hanahan, D., Weinberg, R.A. (2000), Cell 100, 57-70; Hanahan, D., Weinberg, R.A. (2011), Cell 144, 646-674). Oncogenic activation of MAPK pathway is a signature feature of many human cancers, including melanoma, non-small cell lung cancer (NSCLC) and pancreatic cancer (Dhillon, A.S. et al. (2007), Oncogene 26, 3279-3290). For example, 50% of melanomas are caused by the BRAF-V600E oncoprotein which activates constitutive MAPK signalling (Davies, H. et al. (2002), Nature 417, 949-954). BRAF-V600E specific small molecule inhibitors are the standard therapeutic approach for treatment of BRAF-V600E-positive metastatic melanoma. While this treatment leads to dramatic tumor shrinkage in the first few months, almost all patients acquire resistance as treatment continues (Flaherty, K.T. et al. (2010), N. Engl. J. Med. 363, 809-819; Johnson et al. (2015), Eur J Cancer, 51(18):2792-9). It has been shown that progression free survival can be extended by combination therapy, for example by co-targeting BRAF and a second inhibitor, such as the downstream kinase MEK and, accordingly, methods have been developed in WO 2011/112678 A1 and WO 2014/147573 A2, but most patients still acquire resistance-causing genetic alterations limiting the benefit of these molecularly targeted drugs (Flaherty, K.T. et al. (2012), N. Engl. J. Med. 367, 1694-1703; Larkin, J. et al. (2014), N. Engl. J. Med. 371, 1867-1876; Long, G.V. et al. (2014), N. Engl. J. Med. 371, 1877- 1888). Similar clinical hurdles are faced in the treatment of NSCLC with small molecule inhibitors or antibodies targeting EGFR as patients invariably acquire new, resistance causing mutations (Jänne et al. (2005), J. Clin. Oncol., 23:3227-3234; Kobayashi et al. (2005), N Engl J Med, 352:786-792).

Based on the above-mentioned state of the art, the objective of the present invention is to provide means and methods to identify novel compounds able to prevent the development of resistance to inhibitors of the MAPK and EGFR pathway, in particular in a combination medicament with inhibitors of the MAPK and EGFR pathway and/or other regimens where similar drug tolerance mechanisms emerge. This objective is attained by the claims of the present specification.

### Terms and definitions

The term "protein comprised in a signalling pathway" in the context of the present specification relates to molecules that interact in a cell to control a specific cellular function, such as proliferation, differentiation or apoptosis. Molecules that are comprised in one signalling pathway are part of a concerted activation cascade. Upon a stimulus, the first molecule in the pathway activates one or several downstream molecules. The activation is passed on until the last molecule in the activation chain is activated and the cellular function is carried out.

With respect to the MAPK pathway, this relates to specific ligands, receptors and downstream transcription factors selected from the group comprising NGF, NRG, BDNF, NT3/4, EGF, FGF, PDGF, CACN, TrkA/B, EGFR, FGFR, PDGFR, ROS, ALK, MET, KIT, GFB2, SOS, HRAS, KRAS, NRAS, RasGRF, RasGRP, CNasGEF, PKC, PKA, Rap1, G12, Gap1m, NF1, p120GAF, RafB, ARAF, BRAF, CRAF, Mos, MEK1, MEK2, MP1, ERK1, ERK2, PTP, MKP, Tau, STMN1, cPLA2, MNK1/2, RSK2, CREB, Elk-1, Sap1a, c-Myc, SRF and c-fos.

The term "activating mutation" in the context of the present specification relates to an alteration in the nucleotide sequence of a gene that results in an increased activity of the gene product. The increased activity can be due to enhanced enzymatic activity, prolonged half-life or overexpression of the gene product.

The term "gene under transcriptional control of SOX2" in the context of the present specification relates to a gene characterized by a first expression level if SOX2 is not expressed in the same cell, and a second expression level if SOX2 is expressed in the same cell. The first expression level is lower than the second expression level.

In certain embodiments, the gene under transcriptional control of SOX2 is selected from the group comprising *Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1* and *ROBO2.*

The expression level of SOX2 and/or the expression level of said gene under transcriptional control of SOX2 can be determined by analysis of protein expression and/or mRNA expression.

The term "treatment" as used herein means preventing, relieving, reducing or alleviating at least one symptom of a disease. For example, treatment can be the diminishment of one or several symptoms of a disorder or complete eradication of a disorder, such as cancer. Within the meaning of the present invention, the term "treatment" also denotes to arrest, delay the onset (i.e., the period prior to clinical manifestation of a disease) and/or reduce the risk of developing or worsening a disease.

As used herein, the term "melanoma" refers to a form cancer that develops from melanocytes. Melanomas typically affect the skin but may also affect the mouth, intestines, or eye. Melanomas may also spread (metastasize) through the blood or lymph systems to organs and bones. Melanoma may develop from an existing mole or other mark on the skin or from unmarked skin.

The terms "non-small cell lung cancer" and "NSCLC" as used interchangeably herein refer to a malignant neoplasm of the lung which is not of the oat cell (or small cell) type, and includes but is not limited to the recognized types of bronchogenic carcinomas (those arising from the lining of the bronchi) including adenocarcinoma, squamous cell carcinoma, and large cell carcinoma.

As used herein, the term "resistance" and the like includes a non-responsiveness or decreased responsiveness to treatment with an inhibitor. Non-responsiveness or decreased responsiveness may include an absence or a decrease of the benefits of treatment, such as a decrease or cessation of the relief, reduction or alleviation of at least one symptom of the disease.

As used herein, the term "contact" and the like means bringing together, either directly or indirectly, an inhibitor of the signaling pathway and/or a test compound into physical proximity to a cell that carries an activating mutation in a gene encoding a protein comprised in the signaling pathway. Contacting the cells with the inhibitor and/or a test compound may occur in any culture media and under any culture conditions that promote the survival of the cells.Contacting includes, for example, placing the inhibitor of the signaling pathway and/or the test compound into a container, such as a beaker, microtiter plate, cell culture flask, or a microarray, or the like, which contains a cell that carries an activating mutation in a gene encoding a protein comprised in the signaling pathway. Alternatively, by contact it is meant to bring an agent into close proximity with another agent such that both agents can interact with each other. For example an antibody or other binding member may be brought into close proximity with a protein in a cell and where the antibody has binding specificity for the protein the antibody will bind the protein. In yet another example, a first nucleic acid may be brought into close proximity with a second complementary nucleic acid (a primer with a target sequence) and can be incubated such that binding may be detected or amplification of the target sequence may occur.

Furthermore, in the claims the word "comprising" does not exclude other elements or steps, and the indefinite article "a", "an", and "the" include plural referents unless the context clearly dictates otherwise. Thus, for example, reference to "an inhibitor" includes one or more inhibitors.

### Description

According to a first aspect of the invention, a cell-based screening method to identify compounds effective in the treatment of cancer is provided. The cancer is characterized by cells that are characterized by an over-activated signalling pathway. The over-activation is caused by an activating mutation in or amplification of a gene encoding a protein that acts in the signalling pathway. The skilled person is aware that over-activation of a signaling pathway may also be caused by an inhibiting mutation in or a deletion of a gene encoding an inhibitor of the signaling pathway such as NF1. The compounds are effective in the treatment of the cancer in a combination medicament comprising an inhibitor of the respective over-activated signalling pathway. The cell based screening method comprises the following steps:
a. A cell that carries an activating mutation in a gene encoding a protein comprised in the signalling pathway is provided.
b. The cell is brought in contact with an inhibitor of the signalling pathway and a test compound.
c. The expression level of SOX2 and/or of a gene under transcriptional control of SOX2 is determined.
d. A score is assigned to the test compound. The score reflects the efficacy of the test compound.

The score is high if the expression level of SOX2 and/or of a gene under transcriptional control of SOX2, particularly a gene selected from the group consisting of *Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1* and *ROBO2* is below a predetermined threshold. The predetermined threshold corresponds to the expression level of SOX2 and/or of a gene under transcriptional control of SOX2 in a control cell treated solely with the inhibitor of the signalling pathway, but not with the test compound. Accordingly, the score is determined to be high if expression of SOX2 and/or of a gene under transcriptional control of SOX2, e.g. one of the genes listed immediately above, in a control cell that is treated with the inhibitor of the signalling pathway alone, is below expression of SOX2 and/or of the gene under transcriptional control of SOX2 in a cell that has been brought in contact with the inhibitor of the signalling pathway together with the test compound used in the methods of the present invention.

The score is low if the expression level of SOX2 and/or of a gene under transcriptional control of SOX2, particularly a gene selected from the group consisting of *Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1* and *ROBO2* is equal to or above the predetermined threshold. Accordingly, the score is determined to be low if expression of SOX2 and/or of a gene under transcriptional control of SOX2, e.g. one of the genes listed immediately above, in a control cell that is treated with the inhibitor of the signalling pathway alone, is above expression of SOX2 and/or of the gene under transcriptional control of SOX2 in a cell that has been brought in contact with the inhibitor of the signalling pathway together with the test compound used in the methods of the present invention.

In a preferred embodiment of the invention, the score increases with decreased expression of SOX2 and/or of the gene under transcriptional control of SOX2 as compared to other test compounds. That is, the more expression of SOX2 and/or of the gene under transcriptional control of SOX2 is reduced as compared to the control cell defined above, the higher the score will be. Similarly, the score decreases with increased expression of SOX2 and/or of the gene under transcriptional control of SOX2 as compared to other test compounds. That is, the more expression of SOX2 and/or of the gene under transcriptional control of SOX2 is increased as compared to the control cell defined above, the lower the score will be.

In certain embodiments, steps a to d are performed simultaneously with a plurality of cells.

In certain embodiments,
a. a plurality of cells is employed simultaneously in step a;
b. the plurality of cells is submitted to step b together;
c. the average expression level of SOX2 and/or of a gene under transcriptional control of SOX2, particularly a gene selected from the group consisting of *Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1* and *ROBO2,* is determined simultaneously in the plurality of cells;
d. a high score is assigned to the test compound if the average expression level of SOX2 and/or of said gene under transcriptional control of SOX2 is below the average expression level of SOX 2 and/or of a gene under transcriptional control of SOX2 in control cells treated solely with the inhibitor of the signalling pathway.

In certain embodiments,
a. a plurality of cells is employed simultaneously in step a;
b. the plurality of cells is submitted to step b together;
c. a single cell is evaluated as "SOX2 positive" if the expression level of SOX2 and/or of said gene under transcriptional control of SOX2 is above an expression level determined for an untreated cell and a ratio of "SOX2 positive" cells to total cells is determined for said plurality of cells; and
d. a high score is assigned to the test compound if the ratio determined for cells treated with the test compound is below the ratio determined for control cells treated solely with the inhibitor of the signalling pathway.

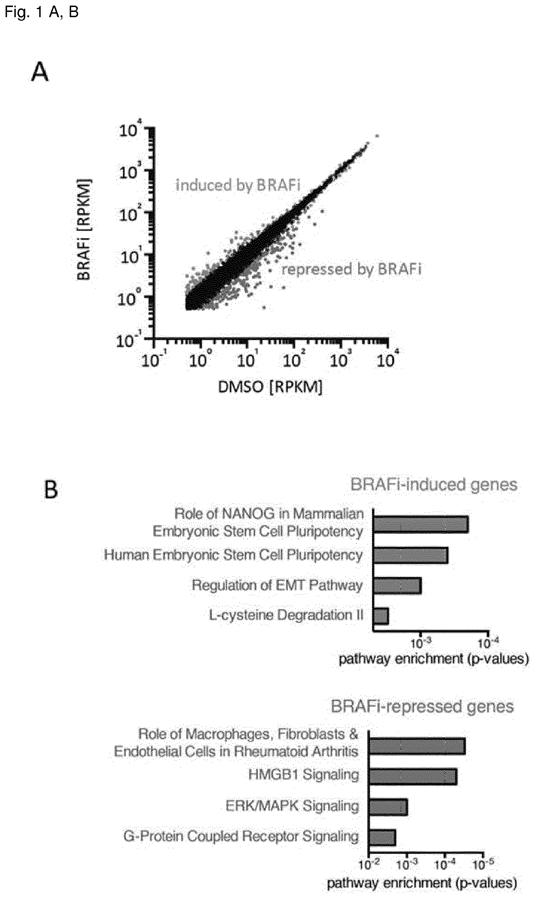

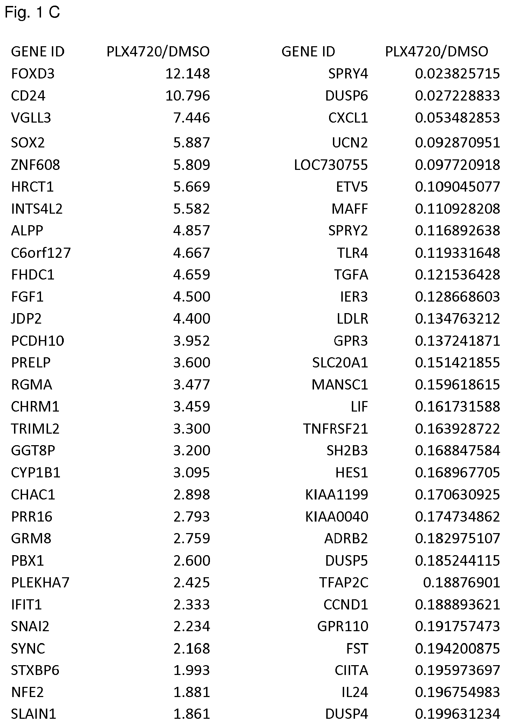

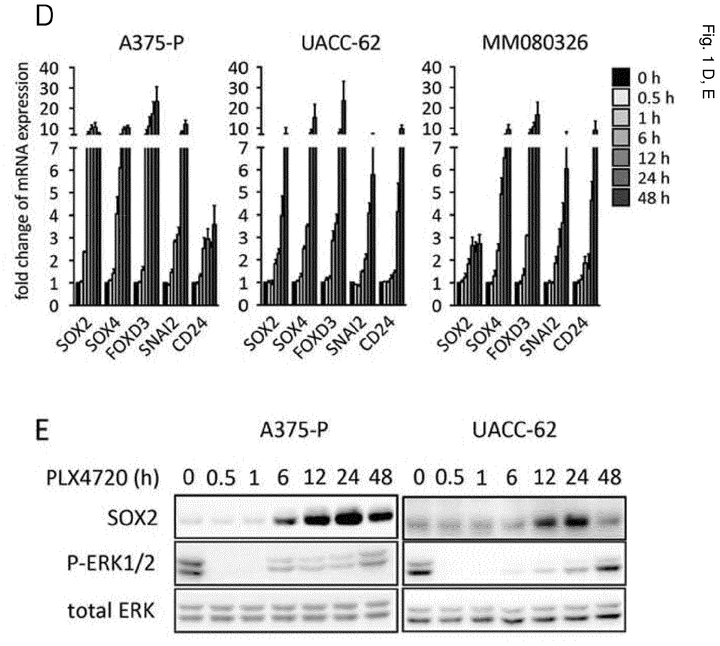

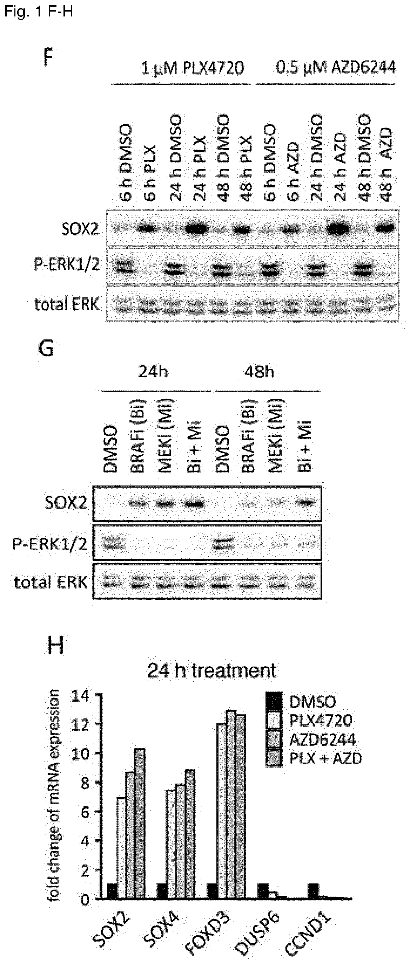

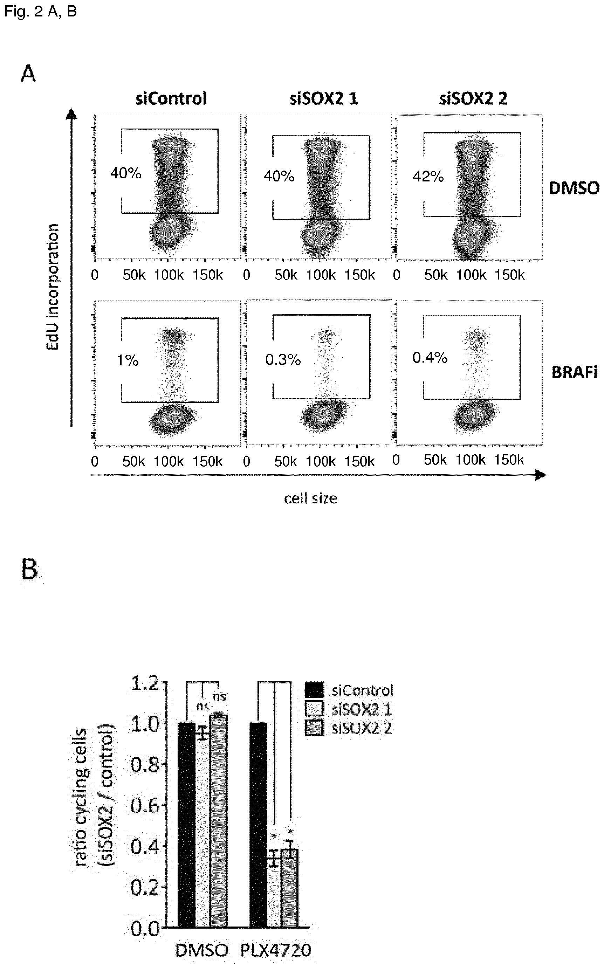

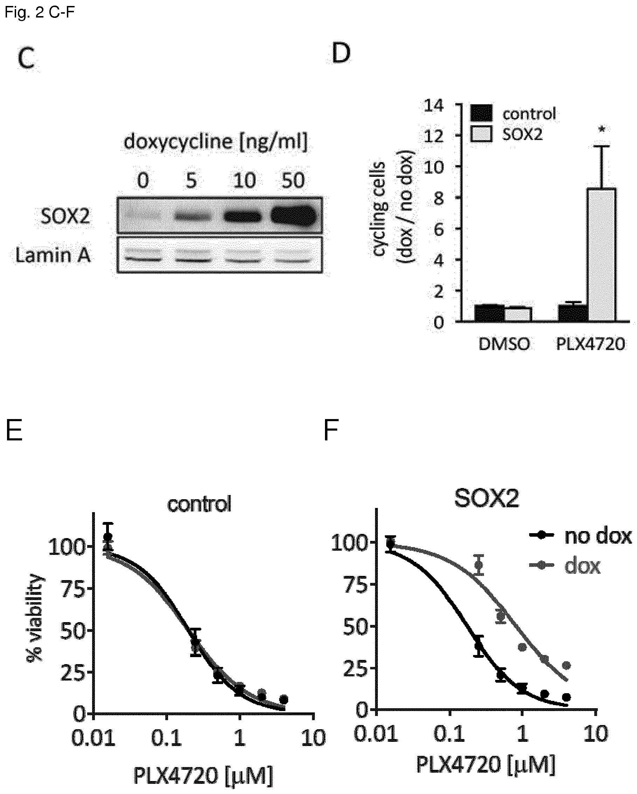

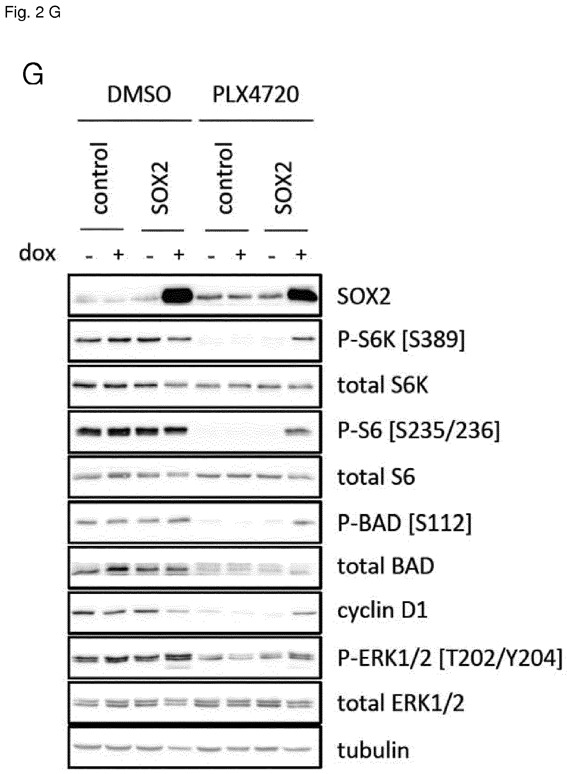

The inventors found that inhibitors of mutant BRAF and MEK (hereafter collectively referred to as MAPK inhibitors, MAPKi) induce an acute transcriptional response (i.e. adaptive resistance programs; ARPs) in melanoma cells within hours of drug application. These ARPs involve the transcriptional induction of SOX2-dependent stemness, axon guidance and EMT (epithelial-to-mesenchymal transition) genes, leading to the generation of a pool of drug-tolerant cells (Figure 1). These drug-tolerant cells can seed the development of acquired resistance in the long-term. The number of drug-tolerant cells can be significantly reduced by siRNA mediated knockdown of SOX2, the master regulator of stemness and main driver of MAPKi-induced ARPs (Figure 2). As a consequence, preventing the induction of ARPs by blunting MAPKi-induced SOX2 in the clinical context will be of highest therapeutic value to prevent acquired drug resistance thus extending the durability of clinical-used MAPK inhibitors.

SRY (sex determining region Y)-box 2, also known as SOX2, is a transcription factor that is essential for maintaining self-renewal, or pluripotency, of undifferentiated embryonic stem cells. The protein is a member of the Sox family of transcription factors, which have been shown to play key roles in many stages of mammalian development. For example, Sox2 controls the branching morphogenesis of the bronchial tree and differentiation of the epithelium of airways in lung development. Under normal conditions, Sox2 is critical for maintaining self-renewal and appropriate proportion of basal cells in adult tracheal epithelium. However, its overexpression gives rise to extensive epithelial hyperplasia and eventually carcinoma in both developing and adult mouse lungs. Moreover, in squamous cell carcinoma, gene amplifications frequently target the 3q26.3 region. The gene for Sox2 lies within this region, which effectively characterizes Sox2 as an oncogene. For example, Sox2 has been identified as an oncogene in gastric cancer (Yajun et al. (2014), J. Cancer Res. Clin. Oncol., 140(7):1117-24). Sox2 is a key upregulated factor in lung squamous cell carcinoma, directing many genes involved in tumor progression. Sox2 overexpression cooperates with loss of Lkb1 expression to promote squamous cell lung cancer in mice. Its overexpression also activates cellular migration and anchorage-independent growth. SOX2 has also been implicated in glioblastoma multiforme, and the downstream targets for SOX2 were recently identified by ChIP-seq and microarray analyses (Fang et al. (2011), BMC Genomics, 6; 12:11). Sox2 expression is also found in high leason grade prostate cancer, and promotes castration-resistant prostate cancer growth. Sox2 has been shown to be relevant in the development of Tamoxifen resistance in breast cancer. Further, OCT4 which interacts with SOX2 has been shown to be upregulated in drug-resistant prostate cancer cells (Fang et al. (2011), Genes Cancer, 1(9): 908-916). SOX2 has also been shown to be transcriptionally induced in cultured NSCLC cell lines when exposed to EGFR inhibitors (Rothenberg, M.S. et al (2015), eLife, vol. 4, 16). Despite this knowledge, there is no cell-based screening method that allows for identification of compounds that prevent the development of resistance to inhibitors of the MAPK and EGFR pathway by determining the expression level of SOX2 and/or the expression level of a gene under transcriptional control of SOX2. This is because the inventors have for the first time surprisingly found that compounds can be found by an in vitro cell-based screening method as presented herein. In certain embodiments of any aspect of the invention, the expression level of SOX2 and/or the expression level of said gene under transcriptional control of SOX2 is determined by analysis of protein expression and/or mRNA expression.

Protein (SOX2 or protein under transcriptional control of SOX2) can be directly visualized and quantified by antibody-mediated staining. mRNA (of *SOX2* or a gene under transcriptional control of SOX2) can be directly visualized by *in situ* hybridization and individual mRNA molecules can be quantified.

Accordingly, within the present invention, gene expression levels may be determined using any technique known in the art, for example methods based on hybridization of polynucleotides (mRNA transcripts), methods based on sequencing polynucleotides or amplifying polynucleotides.

Quantification of mRNA gene transcript may be performed using, without limitation, northern blotting, in situ hybridization, RNAse protease assays, PCR based methods such as reverse transcription polymerase chain reaction (RT-PCR) and real time quantitative PCT qRT- PCR. Alternatively, antibodies with binding specificity to nucleic acid duplexes may be used to determine mRNA levels. Microarray techniques using specific binding members for RNAs of interest, for example cDNA or oligonucleotide probes specific for RNAs of interest or antibodies specific for mRNA of interest wherein the specific binding members are plated or arrayed on a substrate, for example a glass slide or a microchip substrate can be used. The specific binding members may be provided on the substrate at an addressable location and the number of addressable locations can vary from, for example at least three, at least 10, at least 50, at least 100, at least 1000 or at least 10,000 or more. In embodiments the number of addressable locations can vary from less than 1000, less than 100, less than 50, less than 10, or less than 5. In such embodiments, the cell is contacted with the array and the arrayed specific binding members can form detectable interactions with targets in the cell. The interactions may be detected using suitable labels. Where oligonucleotide probes are utilised, under appropriate conditions the oligonucleotide probes can "hybridize" to a target nucleic acid sequence to form base- paired duplexes with nucleic acid molecules that have a complementary base sequence. Hybridization conditions resulting in particular degrees of stringency will vary depending on the nature of the hybridization method and the composition and length of the hybridizing nucleic acid sequences.

Stringent hybridization occurs when a nucleic acid binds a target nucleic acid with minimal background. Typically, to achieve stringent hybridization, temperatures of around 1° C to about 20° C, more preferably 5° C to about 20° C below the Tm (melting temperature at which half the molecules dissociate from their partner) are used. However, it is further defined by ionic strength and pH of the solution. Suitable hybridization conditions would be known to those of skill in the art, and exemplary hybridization conditions are: Very high stringency (detects sequences that share at least 90% identity) - hybridization 5x SSC at 65⁰C for about 16 hours, High stringency (detects sequences that share at least 80% identity) - hybridization 5x-6x SSC at 65⁰C for 16 hours, and Low stringency (detects sequences that share at least 50% identity) - hybridization 6x SSC at room temperature to 55 °C for 20 to 30 minutes.

An example of a highly stringent wash condition is 0.15 M NaCl at 72° C for about 15 minutes. An example of a stringent wash condition is 0.2X sodium chloride and sodium citrate (SSC) wash at 65° C for 15 minutes (see, Sambrook and Russell, infra, for a description of SSC buffer for example 2Ox SSC made by dissolving 175.3g of NaCl and 88.9 g of sodium citrate in 800 ml distilled water. Adjusting pH to pH7.0 with HCI (IM) and adjusting volume to IL with distilled water). Often, a high stringency wash is preceded by a low stringency wash to remove background probe signal. An example of a medium stringency wash for a duplex of, for example, more than 100 nucleotides, is 1X SSC at 45° C for 15 minutes. An example of a low stringency wash for a duplex of, for example more than 100 nucleotides, is 4-6X SSC at 40° C for 15 minutes. For short probes (for example about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30° C and at least about 60° C for long probes (for example, > 50 nucleotides).

The methodology used in PCR methods for example RT-PCR and PCT and RT-PCR will be well known to those skilled in the art.

Some methods may require the isolation of RNA. Such isolation techniques are known in the art and may utilize commercially available RNA isolation kits from manufacturers such as Qiagen.

Determination of protein expression Immunohistochemistry (IHC) and ELISA are techniques useful for detecting protein expression. Antibodies or binding fragments of antibodies (monoclonal or polyclonal) may be used in the disclosed methods and kits. Antibodies can be detected by direct labeling of the antibodies or by using a second antibody which is specific for the primary antibody which has binding specificity for the target. The second antibody can be labeled with a detectable moiety or can be conjugated to a hapten (such as a biotin or the like) wherein the hapten is detectable by a detectably labeled cognate hapten binding molecule, for example streptavidin horseradish peroxidise.

The binding specificity of antibodies (antibodies with binding specificity to a particular antigen, e.g. SOX2 and/or another gene under transcriptional control of SOX2) can be established using Western blotting, in parallel with immunohistochemical analysis of formalin-fixed, paraffin-embedded cell lines mimicking the handling of the primary tumours (as described by O'Brien et al., 2007, International Journal of Cancer, 120:1434-1443)

Alternatively, proteins may be detected using aptamers (for example a single stranded nucleic acid molecule (such as, DNA or RNA) that assumes a specific, sequence dependent shape and binds to FKBPL protein with high affinity and specificity), mirror image aptamers (SPIEGELMER^{™}), engineered nonimmunuoglobulin binding proteins, for example nonimmunoglobulin binding proteins based on scaffolds including fibronectin (ADNECTINS^{™}), CTLA-1 (EVIBODIES^{™}), lipocalins (ANTICALINS^{™}), protein A domain (AFFIBODIES^{™}) or the like. In embodiments, an aptamer may comprise less than 100 nucleotides, less than 75 nucleotides, less than 50 nucleotides, for example 25 to 50 nucleotides, 10 to 50 nucleotides, 10 to 100 nucleotides.

In particular embodiments, an array may be provided comprising protein sequences, including SOX2 protein or fragments of SOX2 protein or antibodies with binding specificity to SOX2 protein or fragments thereof. These protein sequences or antibodies can be conjoined to a substrate. Changes in protein expression can be detected by, for example, measuring the level of SOX2 protein and/or another gene under transcriptional control of SOX2 which binds to antibodies with binding specificity to SOX2 protein and/or another gene under transcriptional control of SOX2 when the cell is brought into contact with the array.

Suitable substrates for use in an array and array formats would be known to those of skill in the art.

In particular embodiments IHC samples can be analysed using an automated image analysis system, so as to provide a blinded analysis. For this, whole-slide digital images can be first captured at 2Ox using a ScanScope XT Slide Scanner (Aperio Technologies). Secondly, a positive pixel count algorithm (Aperio Technologies) can be used to develop a quantitative scoring model for SOX2 expression. Statistical analysis of tissue microarray-derived data can be carried out using the v2 test for trend, Fisher's exact and Mann-Whitney tests for comparison of SOX2 expression and Kaplan-Meier plots can be used for survival analysis and the curves compared using the log-rank test. Cox proportional hazards regression can be used to estimate proportional hazard ratios and conduct multivariate analyses as described previously. All calculations can be performed with SPSS v11.0 (SPSS, IL). In addition, to facilitate generation of discrete multi-marker test, fluorescently-tagged antibodies (carrying non-overlapping fluorophores) and additional relevant biomarkers can be used simultaneously. Advantageously a recently developed fluorescent scanning system from Aperio, for example, the ScanScope FL system could be used. This assay method would provide a further layer of sophistication by providing more quantitative analysis than that afforded by conventional brightfield imaging. As will be appreciated, the methods of detecting the expression of SOX2, the location of SOX2 in a cell or the activity of SOX2 may be applicable in relation to any of the methods of the invention described herein or claimed.

Preferred features and embodiments of each aspect of the invention are as for each of the other aspects mutatis mutandis unless context demands otherwise.

A nucleic acid molecule is said to be complementary with another nucleic acid molecule if the two molecules share a significant number of complementary nucleotides to form a stable duplex or triplex when the strands bind (hybridise) to each other, for example by forming Watson- Crick base pairs. Complementarity can be described as a percentage of the proportion of base pairs between two nucleic acid molecules within a specific region of two molecules.

By detect is meant determining if an interaction between two agents for example two proteins or two nucleic acids is present or absent. This may include quantification. Detection may include the use of an agent which is capable of detection (a label) using for example spectrophotometry, flow cytometry, or microscopy. Exemplary labels include radioactive isotopes (such as ³H, ¹⁴C, ¹⁵N, ³⁵S, ⁹⁰V, ⁹⁹Tc, ¹¹¹Ln, ¹²⁵I₁Or ¹³¹I), fluorophores (such as fluorescein, fluorescein isothiocyanate, rhodamine or the like), chromophores, ligands, chemiluminescent agents, bioluminescent agents (such as luciferase, green fluorescent protein (GFP) or yellow fluorescent protein), enzymes that can produce a detectable reaction product (such as horseradish peroxidise, luciferase, alkaline phosphatase, beta-galactosidase) and combinations thereof.

By specific binding is meant a particular interaction between one binding partner and another binding partner, for example a primer and a target sequence or a protein specific antibody and a protein. Interactions between one binding partner and another binding partner may be mediated by one or more, typically more than one, non-covalent bonds. An exemplary way of characterising specific binding is by a specific binding curve.In certain embodiments, the method comprises a step in which the cell cycle phase is determined in the cell treated with the inhibitor of the signalling pathway and the test compound. In certain embodiments, cell cycle arrest is detected. A high score is assigned to the test compound if the treated cell undergoes cell cycle arrest and does not overcome the cell cycle arrest.

In certain embodiments, the over-activated signalling pathway is the MAPK- or EGFR pathway.

In certain embodiments, the cancer is characterized by cancer cells that carry an activating mutation or amplification in a gene encoding a protein comprised in the MAPK or EGFR pathway.

In certain embodiments, the activating mutation or amplification in the MAPK or EGFR pathway affects *NRAS, KRAS, HRAS, ARAF, BRAF, CRAF, MEK1*/*2, ERK1*/*2, ROS, ALK, MET, KIT* or *EGFR.*

In certain embodiments, the cancer is selected from melanoma, non-small cell lung cancer, prostate cancer, bile duct cancer, bladder cancer, pancreatic cancer, thyroid cancer, ovarian cancer, colorectal tumor, hairy cell leukemia, acute myeloid leukemia, multiple myeloma, liver cancer, breast cancer, esophageal cancer, head and neck cancer and glioma.

In certain embodiments, the cancer is selected from melanoma and non-small cell lung cancer.

In certain embodiments, the cell characterized by an activating mutation in or amplification of a gene encoding a protein that acts in the over-activated signalling pathway and choosen for the screening method is selected from a melanoma cell or a non-small cell lung cancer cell carrying a *BRAF* mutation and a non-small cell lung cancer cell carrying a *EGFR* mutation, amplification or overexpression.

In certain embodiments, the *BRAF* mutation comprises the BRAF-V600E or BRAF-V600K mutation. Both mutations are characterized by a substitution of valine at codon 600 with glutamate or lysine, respectively, and affect the kinase domain of the enzyme.

In certain embodiments, the inhibitor of the over-activated signalling pathway is selected from an inhibitor of the EGFR pathway (EGFRi) and an inhibitor of the MAPK pathway (MAPKi),

In certain embodiments of any aspect of the invention the MAPKi is selected from an inhibitor of B-Raf (BRAFi), an inhibitor of MEK (MEKi), and an inhibitor of ERK (ERKi).

In certain embodiments, the BRAFi is selected from the group comprising vemurafenib (CAS No. 1029872-54-5.), dabrafenib (CAS No. 1195765-45-7), encorafenib (CAS No. 1269440-17-6), LGX818 (CAS No. 1269440-17-6), PLX4720 (CAS No. 918505-84-7), TAK-632 (CAS No. 1228591-30-7), MLN2480 (CAS No. 1096708-71-2), SB590885 (CAS No. 405554-55-4*)*, XL281 (BMS-908662, CAS No. 1029873-02-6, CAS No. 870603-16-0) and RAF265 (CAS No. 927880-90-8).

In certain embodiments, the MEKi is selected from the group comprising AZD6244 (CAS No. 606143-52-6), trametinib (CAS No. 871700-17-3), selumetinib (CAS No. 606143-52-6), cobimetinib (CAS No. 934660-93-2), binimetinib (CAS No. 606143-89-9), MEK162 (CAS No. 606143-89-9), RO5126766 (CAS No. 946128-88-7), GDC-0623 (CAS No. 1168091-68-6), PD 0325901 (CAS No. 391210-10-9), CI-1040 (CAS No. 212631-79-3) and TAK-733 (CAS No. 1035555-63-5).

In certain embodiments, the ERKi is selected from the group comprising ulixertinib (CAS No. 869886-67-9), SCH772984 (CAS No. 942183-80-4), XMD8-92 (CAS No. 1234480-50-2), FR 180204 (CAS No. 865362-74-9), GDC-0994 (CAS No. 1453848-26-4), ERK5-IN-1 (CAS No. 1435488-37-1), DEL-22379 (CAS No. 181223-80-3), BIX 02189 (CAS No. 1265916-41-3, 1094614-85-3), ERK inhibitor (CAS No. 1049738-54-6), ERK inhibitor III (CAS No. 331656-92-9), GDC-0994 (CAS No. 1453848-26-4) and VTX11e (CAS No. 896720-20-0).

In certain embodiments, the EGFRi is selected from the group comprising cetuximab (CAS No. 205923-56-4), panitumumab (CAS No. 339177-26-3), zalutumumab (CAS No. 667901-13-5), nimotuzumab (h-R3, BIOMAb EGFR, TheraCIM, Theraloc, CIMAher, CAS No. 828933-51-3), matuzumab (CAS No. 339186-68-4), gefitinib (CAS No. 184475-35-2), erlotinib (CAS No. 183321-74-6), lapatinib (Tykerb, Tyverb, CAS No. 231277-92-2, CAS No. 388082-78-8), AP26113 (Brigatinib, CAS No. 1197953-54-0), EGFR inhibitor (CAS No. 879127-07-8), EGFR/ErbB-2/ErbB-4 Inhibitor (CAS No. 881001-19-0), EGFR/ErbB-2 Inhibitor (CAS No. 179248-61-4), EGFR inhibitor II (BIBX 1382,CAS No. 196612-93-8), EGFR inhibitor III (CAS No. 733009-42-2), EGFR/ErbB-2/ErbB-4 Inhibitor II (CAS No. 944341-54-2) and PKCβII/EGFR Inhibitor (CAS No. 145915-60-2).

The test compound with which cells are treated together with the inhibitor of the signaling pathway inhibits the expression level of SOX2 and/or of a gene under transcriptional control of SOX2. In a preferred embodiment the second chemical substance inhibits a gene selected from the group consisting of SOX2, Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1 and ROBO2. It is preferred that the test compound inhibits the expression of SOX2.

Said test compounds include, but are not limited to, small molecule compounds, nucleid acids, siRNAs, mirRNAs, shRNAs, proteins, peptides, antibodies, antibody fragments, aptamers, carbohydrates and lipids. Such test compound can be natural or synthetic.

Test compounds may be obtained from a wide variety of sources including libraries of synthetic or natural compounds. In certain embodiments, a library of test compounds can be screened with the method of the invention. Examples include but are not limited to a peptide library, a nucleic acid library, a peptide nucleic acid library, and a small molecule library. In certain embodiments, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries

The methods for identifying compounds that prevent the development of resistance to inhibitors of the MAPK and EGFR pathway as described herein may also be applied to high throughput screening. High throughput screening (HTS) technology is commonly used to define the rapid processing of cells on a large scale. In certain embodiments, a plurality of screens may be run in parallel with different test compound concentrations to obtain a differential response to the various concentrations. As known in the art, determining the effective concentration of an agent typically uses a range of concentrations resulting from 1:10, or other log scale, dilutions. The concentrations may be further refined with a second series of dilutions, if necessary. Typically, one of these concentrations serves as a negative control, i.e. at zero concentration or below the level of detection of the agent or at or below the concentration of agent that does not give a detectable change in the phenotype. In some instances, a positive control may also be run.

The invention is further illustrated by the following figures, from which further embodiments and advantages can be drawn. These examples are meant to illustrate the invention but not to limit its scope.

### Brief description of the figures

Fig. 1 shows the induction of a stemness and EMT signature upon acute treatment with MAPKi analysed by RNA-Seq. (A) A375-P cells were treated with PLX4720 (BRAFi) or DMSO for 6 h, RNA was isolated and RNA-Seq was performed. Scatter plot shows all genes with minimal normalized abundance of ≥ 0.5 RPKM. Differentially expressed genes (DEGs) selected for pathway analysis are represented by red and blue dots depending on the direction of the transcriptional change (red genes are induced upon PLX4720, blue genes are repressed upon PLX4720). (B) Bar graphs showing the five top pathways with significant enrichment of the analyzed DEGs. Longer bars indicate stronger significance. (C) Top deregulated genes upon treatment with PLX4720 (left column). Top genes induced and repressed by PLX4720 are shown in separate lists. (D) Indicated cell lines were treated with 1 µM PLX4720 or 0.01% DMSO (0 h) for the indicated times and the expressional changes of selected genes associated with stemness/EMT were confirmed by qPCR. All cell lines expressed similar adaptive gene programs upon BRAF inhibition. Data shown represent mean of independent biological triplicates ± SEM. (E) Western blot analysis of the same experiment as shown in (D). As key player in the stemness signature, SOX2 levels were assessed. P-ERK1/2 levels served as positive treatment controls. Total ERK1/2 levels served as loading control. (F) A375-P cells were treated with 1 µM PLX4720, 0.5 µM AZD6244 (MEK1/2 inhibitor) or 0.01% DMSO for the indicated times and SOX2 levels were assessed by Western blot analysis. Note that inhibition of BRAF and inhibition of MEK1/2 induce similar SOX2 levels, suggesting this to be a general feature of oncogenic MAPK inhibition. (G) A375-P cells were treated with 1 µM PLX4720 (BRAFi), 0.5 µM AZD6244 (MEKi), a combination of both drugs or 0.01% DMSO for the indicated times and SOX2 levels were assessed by Western blot analysis. Note that BRAFi and MEKi had comparable effects on SOX2 levels, with the combination of both drugs leading to slightly higher SOX2 levels. (H) A375-P cells were treated with 1 µM PLX4720 (BRAFi), 0.5 µM AZD6244 (MEKi), a combination of both drugs or 0.01% DMSO for the 24 h and the expressional changes of selected genes associated with stemness/EMT were confirmed by qPCR. Note that BRAFi and MEKi had comparable effects on transcript levels, with the combination of boths drugs being slightly more efficient at transcript induction. For all Western blots, individual time points were assessed in at least two independent experiments. Representative data is shown.
Fig. 2 shows the protective effect of SOX2 from MAPKi-induced anti-proliferative effects. (A) A375-P cells were transfected with 100 nM siRNA targeting SOX2 or a negative control siRNA. Cells were then treated with PLX4720 (BRAFi) or DMSO for 48 h. 2 h before harvesting, cells were pulsed with 3 uM EdU to label cycling cells. EdU-positive cells were detected using flow cytometry. Note that SOX2 knockdown has no effect on cell proliferation when BRAF is active (DMSO) but reduces the pool of drug-tolerant, cycling cells in the BRAFi condition. Representative data is shown. (B) Quantification of experiment (A). For each condition, percentage of cycling cells upon siRNA knockdown of SOX2 was normalized to the negative control siRNA. Data shown represent mean of independent biological triplicates ± SEM. (C) A375-P cells stably transduced with pTRIPZ-SOX2 vector were treated with the indicated concentrations of doxycycline for three days or with 1 µM PLX4720 (PLX) for 24 h. SOX2 overexpression was confirmed by Western blot analysis. Lamin A served as loading control. Individual concentrations were assessed in at least two independent experiments. Representative results are shown. (D) A375-P/pTRIPZ-control or -SOX2 cells were pre-treated with 0 ng / ml (negative control) or with 50 ng / ml doxycycline for 24 h, and subsequently with DMSO or PLX4720 for an additional 48 h. 2 h before harvesting, cells were pulsed with 3 uM EdU to label cycling cells. EdU-positive cells were detected using flow cytometry. Data shown is normalized to the percentage of cycling cells in the no doxycycline condition. Shown is mean of independent biological triplicates ± SEM. (E-F) A375-P/pTRIPZ-control or -SOX2 cells were pre-treated with 0 ng / ml (negative control) or with 50 ng / ml doxycycline for 24 h, and subsequently with serial dilutions of PLX4720 for an additional four days. Cell viability was assessed by PrestoBlue assay and IC50 values were calculated. Note that doxycycline has no effect on pTRIPZ-control cells but shifts the survival curve to the right in the pTRIPZ-SOX2 cells, indicating decreased drug sensitivity when SOX2 is overexpressed. IC50 values are stated above the survival curves. Data shown is normalized to DMSO-treated cells. Error bars indicate SEM of three independent experiments. (G) A375-P/pTRIPZ-control or -SOX2 cells were pre-treated with 0 ng / ml (negative control) or with 50 ng / ml doxycycline for 24 h, and subsequently with DMSO or PLX4720 for an additional 48 h. Survival signaling was assessed by Western blot. Survival signaling via S6K and BAD is repressed by PLX4720 but rescued when SOX2 is overexpressed by doxycycline treatment.

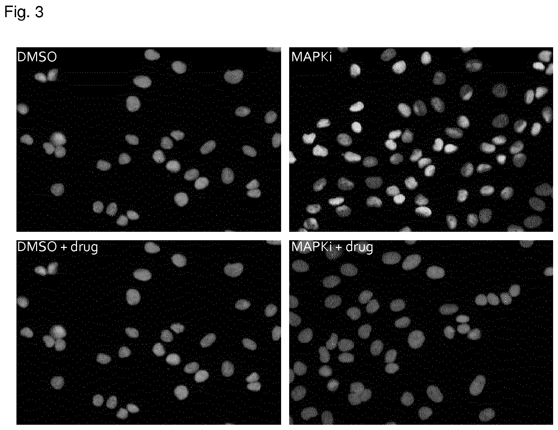

Fig. 3 shows the Readout of the Modular Screening Approach. Immunofluorescence staining of SOX2 in BRAF^{V600E}-mutated human melanoma cells.

## Claims

1. An in vitro cell-based screening method to identify compounds effective in the treatment of cancer **characterized by** cells that are **characterized by** an over-activated signalling pathway, in a combination medicament with an inhibitor of said signalling pathway comprising the steps of
a) providing a cell that carries an activating mutation or amplification in a gene encoding a protein comprised in said signalling pathway;
b) bringing said cell in contact with
i. an inhibitor of said signalling pathway; and
ii. a test compound;
c) determining an expression level of SOX2 and/or a gene under transcriptional control of SOX2; and
d) assigning to said test compound a score, wherein
i. said score is high if said expression level of SOX2 and/or said gene under transcriptional control of SOX2 is below a predetermined threshold, said threshold corresponding to an expression level of SOX2 and/or of said gene under transcriptional control of SOX2 in a control cell treated solely with said inhibitor of said signalling pathway;
ii. said score is low if said expression level of SOX2 and/or of said gene under transcriptional control of SOX2 is equal to or above said predetermined threshold;
wherein the gene under transcriptional control of SOX2 is selected from the group consisting of Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1 and ROBO2.

2. The method according to claim 1, wherein
a) a plurality of cells is employed simultaneously in step a;
b) said plurality of cells is submitted to step b together;
c) the average expression level of SOX2 and/or of said gene under transcriptional control of SOX2 is determined for said plurality of cells; and
d) a high score is assigned to said test compound if said average expression level of SOX2 and/or of said gene under transcriptional control of SOX2 is below the average expression level of SOX2 and/or of said gene under transcriptional control of SOX2 in control cells treated solely with said inhibitor of said signalling pathway.

3. The method according to claim 1, wherein
a. a plurality of cells is employed simultaneously in step a;
b. said plurality of cells is submitted to step b together;
c. a single cell is evaluated as "SOX2 positive" if said SOX2 expression level and/or the expression level of said gene under transcriptional control of SOX2 is above an expression level determined for an untreated cell and a ratio of "SOX2 positive" cells to total cells is determined for said plurality of cells; and
d. a high score is assigned to said test compound if said ratio determined for cells treated with said test compound is below said ratio determined for control cells treated solely with inhibitor of said signalling pathway.

4. The method according to any one of the above claims, wherein said SOX2 expression level and/or the expression level of said gene under transcriptional control of SOX2 is determined by analysis protein expression and/or mRNA expression.

5. The method according to any one of the above claims, comprising a step wherein cell cycle phase is determined, in particular cell cycle arrest is detected, in said cell treated with said inhibitor of said signalling pathway and said test compound, and wherein a high score is assigned to said test compound if said cell undergoes cell cycle arrest.

6. The method according to any one of the above claims, wherein said signalling pathway is the MAPK or EGFR pathway.

7. The method according to claim 6, wherein said cancer is **characterized by** cancer cells that carry an activating mutation or amplification in a gene encoding a protein comprised in the MAPK or EGFR pathway, in particular an activating mutation or amplification in NRAS, KRAS, HRAS, ARAF, BRAF, GRAF, MEK1/2, ERK1/2, ROS, ALK, MET, KIT or EGFR.

8. The method according to any one of claims 6 or 7, wherein said cancer is selected from melanoma, non-small cell lung cancer, prostate cancer, bile duct cancer, bladder cancer, pancreatic cancer, thyroid cancer, ovarian cancer, colorectal tumor, hairy cell leukemia, acute myeloid leukemia, multiple myeloma, liver cancer, breast cancer, esophageal cancer, head and neck cancer and glioma, in particular melanoma and non-small cell lung cancer.

9. The method according to any one of claims 6 to 8, wherein said cell provided in step a is selected from a melanoma cell or a non-small cell lung cancer cell carrying a BRAF mutation, in particular the BRAF-V600E or BRAF-V600K mutation, and a non-small cell lung cancer cell carrying a EGFR mutation, amplification or overexpression.

10. The method according to any one of claims 6 to 9, wherein said inhibitor of said signalling pathway is selected from an inhibitor of the EGFR pathway (EGFRi) and an inhibitor of the MAPK pathway (MAPKi), wherein in particular said MAPKi is selected from an inhibitor of B-Raf (BRAFi), an inhibitor of MEK (MEKi), and an inhibitor of ERK (ERKi).

11. The method according to claim 10, wherein
said BRAFi is selected from the group comprising vemurafenib, dabrafenib,encorafenib, LGX818, PLX4720, TAK-632, MLN2480, SB590885, XL281 andRAF265,
said MEKi is selected from the group comprising AZD6244, trametinib, selumetinib, cobimetinib, binimetinib, MEK162, RO5126766, GDC-0623, PD 0325901, CI-1040 and TAK-733,
said ERKi is selected from the group comprising ulixertinib, SCH772984, XMD8-92, FR 180204, GDC-0994, ERK5-IN-1, DEL-22379, BIX 02189, ERK inhibitor (CAS No. 1049738-54-6), ERK inhibitor III (CAS No. 331656-92-9), GDC-0994 and VTX11e, or
said EGFRi is selected from the group comprising cetuximab, panitumumab, zalutumumab, nimotuzumab, matuzumab, gefitinib, erlotinib, lapatinib, AP26113, EGFR inhibitor (CAS No. 879127-07-8), EGFR/ErbB-2/ErbB-4 Inhibitor (CAS No. 881001-19-0), EGFR/ErbB-2 Inhibitor (CAS No. 179248-61-4), EGFR inhibitor II (BIBX 1382,CAS No. 196612-93-8), EGFR inhibitor III (CAS No. 733009-42-2), EGFR/ErbB-2/ErbB-4 Inhibitor II (CAS No. 944341-54-2) and PKCβII/EGFR Inhibitor (CAS No. 145915-60-2).

## Patentansprüche

1. Ein in-vitro zellbasiertes Screening-Verfahren zur Identifikation von Verbindungen, die wirksam sind in der Behandlung von Krebs, der durch Zellen charakterisiert ist, die durch einen überaktivierten Signalweg charakterisiert sind, in einem Kombinationsmedikament mit einem Inhibitor des Signalwegs umfassend die Schritte
a) Bereitstellen einer Zelle, die eine aktivierende Mutation oder Amplifikation in einem Gen trägt, das für ein Protein kodiert, das im Signalweg umfasst ist;
b) In-Kontakt-bringen der Zelle mit
i. einem Inhibitor des Signalwegs; und
ii. einer Testverbindung;
c) Bestimmen des Expressionslevels von SOX2 und/oder einem Gen unter transkriptioneller Kontrolle von SOX2; und
d) Zuordnen eines Scores zu der Testverbindung, wobei
i. der Score hoch ist, wenn der Expressionslevel von SOX2 und/oder dem Gen unter transkriptioneller Kontrolle von SOX2 unterhalb eines vorher bestimmten Schwellenwerts ist, wobei der Schwellenwert dem Expressionslevel von SOX2 und/oder dem Gen unter transkriptioneller Kontrolle von SOX2 in einer Kontrollzelle entspricht, die ausschließlich mit dem Inhibitor des Signalwegs behandelt wurde;
ii. der Score niedrig ist, wenn der Expressionslevel von SOX2 und/oder dem Gen unter transkriptioneller Kontrolle von SOX2 dem Schwellenwert entspricht oder oberhalb des vorher bestimmten Schwellenwerts liegt;
wobei das Gen unter transkriptioneller Kontrolle von SOX2 ausgewählt ist aus der Gruppe bestehend aus Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1 und ROBO2*.*

2. Das Verfahren nach Anspruch 1, wobei
a) eine Vielzahl an Zellen gleichzeitig in Schritt a eingesetzt wird;
b) die Vielzahl an Zellen gemeinsam Schritt b unterzogen wird;
c) der Durchschnittsexpressionslevel von SOX2 und/oder des Gens unter transkriptioneller Kontrolle von SOX2 für die Vielzahl an Zellen bestimmt wird;
und
d) der Testverbindung ein hoher Score zugeordnet wird, wenn der Durchschnittsexpressionslevel von SOX2 und/oder des Gens unter transkriptioneller Kontrolle von SOX2 unter dem Durchschnittsexpressionslevel von SOX2 und/oder des Gens unter transkriptioneller Kontrolle von SOX2 in Kontrollzellen liegt, die ausschließlich mit dem Inhibitor des Signalwegs behandelt wurden.

3. Das Verfahren nach Anspruch 1, wobei
a) eine Vielzahl an Zellen gleichzeitig in Schritt a eingesetzt wird;
b) die Vielzahl an Zellen gemeinsam Schritt b unterworfen wird;
c) eine einzelne Zelle als "SOX2 positiv" bewertet wird, wenn der SOX2 Expressionslevel und/oder der Expressionslevel des Gens unter transkriptioneller Kontrolle von SOX2 über dem Expressionslevel liegt, der für eine unbehandelte Zelle bestimmt wurde und ein Verhältnis von "SOX2 positiven" Zellen zu gesamten Zellen für die Vielzahl an Zellen bestimmt wird; und
d) der Testverbindung ein hoher Score zugeordnet wird, wenn das Verhältnis, das für die Zellen bestimmt wurde, die mit der Testverbindung behandelt wurden, unter dem Verhältnis liegt, das für die Kontrollzellen bestimmt wurde, die ausschließlich mit dem Inhibitor des Signalwegs behandelt wurden.

4. Das Verfahren nach einem der vorstehend genannten Ansprüche, wobei der SOX2 Expressionslevel und/oder der Expressionslevel des Gens unter transkriptioneller Kontrolle von SOX2 durch die Analyse der Proteinexpression und/oder mRNA Expression bestimmt wird.

5. Das Verfahren nach einem der vorstehend genannten Ansprüche, umfassend einen Schritt, wobei die Zellzyklusphase bestimmt wird, insbesondere der Zellzyklusarrest festgestellt wird, in der Zelle, die mit dem Inhibitor des Signalwegs und der Testverbindung behandelt wurde, und in dem ein hoher Score der Testverbindung zugeordnet wird, wenn die Zelle einen Zellzyklusarrest durchläuft.

6. Das Verfahren nach einem der vorstehend genannten Ansprüche, wobei der Signalweg der MAPK oder EGFR Signalweg ist.

7. Das Verfahren nach Anspruch 6, wobei der Krebs durch Krebszellen gekennzeichnet ist, die eine aktivierende Mutation oder Amplifikation in einem Gen tragen, das für ein Protein kodiert, das im MAPK oder EGFR Signalweg umfasst ist, insbesondere eine aktivierende Mutation oder Amplifikation in NRAS, KRAS, HRAS, ARAF, BRAF, GRAF, MEK1/2, ERK1/2, ROS, ALK, MET, KIT oder EGFR.

8. Das Verfahren nach einem der Ansprüche 6 oder 7, wobei der Krebs ausgewählt ist aus Melanom, nicht-kleinzelligem Lungenkrebs, Prostatakrebs, Gallengangskrebs, Blasenkrebs, Bauchspeicheldrüsenkrebs, Schilddrüsenkrebs, Eierstockkrebs, kolorektalem Karzinom, Haarzell-Leukämie, akuter myeloischer Leukämie, multiplem Myelom, Leberkrebs, Brustkrebs, Speiseröhrenkrebs, Kopf-Hals-Karzinom und Gliom, insbesondere Melanom und nicht-kleinzelligem Lungenkrebs.

9. Das Verfahren nach einem der Ansprüche 6 bis 8, wobei die in Schritt a bereitgestellte Zelle ausgewählt ist aus einer Melanomzelle oder einer nicht-kleinzelligen Lungenkrebszelle, die eine BRAF Mutation tragen, insbesondere die BRAF-V600E oder BRAF-V600K Mutation, und einer nicht-kleinzelligen Lungenkrebszelle, die eine EGFR Mutation, Amplifikation oder Überexpression trägt.

10. Das Verfahren nach einem der Ansprüche 6 bis 9, wobei der Inhibitor des Signalwegs ausgewählt ist aus einem Inhibitor des EGFR Singalwegs (EGFRi) und einem Inhibitor des MAPK Signalwegs (MAPKi), wobei insbesondere der MAPKi ausgewählt ist aus einem Inhibitor von B-Raf (BRAFi), einem Inhibitor von MEK (MEKi) und einem Inhibitor von ERK (ERKi).

11. Das Verfahren nach Anspruch 10, wobei
BRAFi ausgewählt ist aus der Gruppe umfassend vemurafenib, dabrafenib, encorafenib, LGX818, PLX4720, TAK-632, MLN2480, SB590885, XL281 und RAF265,
MEKi ausgewählt ist aus der Gruppe umfassend AZD6244, trametinib, selumetinib, cobimetinib, binimetinib, MEK162, RO5126766, GDC-0623, PD 0325901, CI-1040 und TAK-733,
ERKi is ausgewählt ist aus der Gruppe umfassend ulixertinib, SCH772984, XMD8-92, FR 180204, GDC-0994, ERK5-IN-1, DEL-22379, BIX 02189, ERK Inhibitor (CAS No. 1049738-54-6), ERK Inhibitor III (CAS No. 331656-92-9), GDC-0994 und VTX11e, oder
EGFRi ausgewählt ist aus der Gruppe umfassend cetuximab, panitumumab, zalutumumab, nimotuzumab, matuzumab, gefitinib, erlotinib, lapatinib, AP26113, EGFR inhibitor (CAS No. 879127-07-8), EGFR/ErbB-2/ErbB-4 Inhibitor (CAS No. 881001-19-0), EGFR/ErbB-2 Inhibitor (CAS No. 179248-61-4), EGFR Inhibitor II (BIBX 1382,CAS No. 196612-93-8), EGFR Inhibitor III (CAS No. 733009-42-2), EGFR/ErbB-2/ErbB-4 Inhibitor II (CAS No. 944341-54-2) und PKCßII/EGFR Inhibitor (CAS No. 145915-60-2).

## Revendications

1. Procédé de criblage à base de cellule in vitro pour identifier des composés efficaces
dans le traitement du cancer **caractérisé par** des cellules qui sont **caractérisées par** une voie de signalisation hyperactive, dans un médicament de combinaison avec un inhibiteur de ladite voie de signalisation comprenant les étapes consistant à :
a) fournir une cellule qui porte une mutation d'activation ou une amplification dans un gène codant pour une protéine comprise dans ladite voie de signalisation ;
b) mettre ladite cellule en contact avec
i. un inhibiteur de ladite voie de signalisation ; et
ii. un composé test
c) déterminer un niveau d'expression de SOX2 et/ou d'un gène sous le contrôle transcriptionnel de SOX2 ;
et
d) assigner audit composé test un score, dans lequel
i. ledit score est élevé si le niveau d'expression de SOX2 et/ou dudit gène sous le contrôle transcriptionnel de SOX2 est au-dessous d'un seuil prédéterminé, ledit seuil correspondant à un niveau d'expression de SOX2 et/ou dudit gène sous le contrôle transcriptionnel de SOX2 dans une celule témoin traitée seulement avec ledit inhibiteur de ladite voie de signalisation ;
ii. ledit score est faible si ledit niveau d'expression de SOX2 et/ou dudit gène sous le contrôle transcriptionnel de SOX2 est inférieur ou égal audit seuil prédéterminé ;
dans lequel le gène sous le contrôle transcriptionnel de SOX2 est choisi dans le groupe constitué par Nanog, OCT4, FGF4, FBX15, FOXP4, KLF9, CD24, CD271, CD36, ITLN2, TNFSF12, NOX3, CLEC7A, ACYAP1, UNC5C, UNC5D, MUC16, VAV3, FOXD3, VGLL3, ALPP, C3, F2R, ENPP2, ETV4, NTNG1, NTRK2, ROBO1, et ROBO2.

2. Procédé selon la revendication 1, dans lequel
a) une pluralité de cellules est employée simultanément dans l'étape a ;
b) ladite pluralité de cellules est soumise à l'étape b ensemble ;
c) le niveau d'expression moyen de SOX2 et/ou dudit gène sous le contrôle transcriptionnel de SOX2 est déterminé pour ladite pluralité de cellules ; et
d) un score élevé est assigné audit composé test si ledit niveau moyen d'expression de SOX2 et/ou dudit gène sous le contrôle transcriptionnel de SOX2 est au-dessous du niveau d'expression moyen de SOX2 et/ou dudit gène sous le contrôle transcriptionnel de SOX2 dans des cellules témoins traitées seulement avec ledit inhibiteur de ladite voie de signalisation.

3. Procédé selon l'étape 1, dans lequel
a. une pluralité de cellules est employée simultanément dans l'étape a ;
b. ladite pluralité de cellules est soumise à l'étape b ensemble ;
c. une seule cellule est évaluée comme « positive pour SOX2 » si ledit niveau d'expression de SOX2 et/ou le niveau d'expression du gène sous le contrôle transcriptionnel de SOX2 est au-dessus d'un niveau d'expression déterminé pour une cellule non traitée et un rapport de cellules « positives pour SOX2 » aux cellules totales est déterminé pour ladite pluralité de cellules ; et
d. un score élevé est assigné audit composé test si ledit rapport déterminé pour les cellules traitées avec ledit composé test est au-dessous dudit rapport déterminé pour les cellules témoins traitées seulement avec un inhibiteur de ladite voie de signalisation.

4. Procédé selon l'une quelconque des revendications précédentes, dans lequel ledit niveau d'expression de SOX2 et/ou le niveau d'expression dudit gène sous le contrôle transcriptionnel de SOX2 est déterminé par l'analyse de l'expression de protéines et/ou de l'expression d'ARNm.

5. Procédé selon l'une quelconque des revendications précédentes, comprenant une étape dans laquelle la phase du cycle cellulaire est déterminée, en particulier l'arrêt du cycle cellulaire est détecté dans ladite cellule traitée avec ledit inhibiteur de ladite voie de signalisation et ledit composé test, et dans lequel un score élevé est assigné audit composé test si ladite cellule subit un arrêt du cycle cellulaire.

6. Procédé selon l'une quelconque des revendications ci-dessus, dans lequel ladite voie de signalisation est la voie de MAPK ou d'EGFR.

7. Procédé selon la revendication 6, dans lequel ledit cancer est **caractérisé par** des cellules cancéreuses qui portent une mutation d'activation ou une amplification dans un gène codant pour une protéine comprise dans la voie de MAPK ou d'EGFR, en particulier une mutation d'activation ou une amplification dans NRAS, KRAS, HRAS, ARAF, BRAF, GRAF, MEK1/2, ERK1/2, ROS, ALK, MET, KIT ou EGFR.

8. Procédé selon l'une quelconque des revendications 6 ou 7, dans lequel ledit cancer est choisi parmi le mélanome, le cancer du poumon à non petites cellules, le cancer de la prostate, le cancer du canal biliaire, le cancer de la vessie, le cancer pancréatique, le cancer de la thyroïde, le cancer des ovaires, la tumeur colorectale, le leucémie des cellules pileuses, la leucémie myéloïde aiguë, le myélome multiple, le cancer du foie, le cancer du sein, le cancer œsophagien, le cancer de la tête et du cou et le gliome, en particulier le mélanome et le cancer du poumon à non petites cellules.

9. Procédé selon l'une quelconque des revendications 6 à 8, dans lequel ladite cellule fournie dans l'étape a est choisie parmi une cellule de mélanome ou une cellule de cancer du poumon à non petites cellules portant une mutation de BRAF, en particulier la mutation BRAF-V600E ou BRAF-V600K, et une cellule de cancer du poumon à non petites cellules portant une mutation, une amplification ou une surexpression d'EGFR.

10. Procédé selon l'une quelconque des revendications 6 à 9, dans lequel ledit inhibiteur de ladite voie de signalisation est choisi parmi un inhibiteur de la voie d'EGFR (EGFRi) et un inhibiteur de la voie de MAPK (MAPKi), dans lequel en particulier ledit MAPKi est choisi parmi un inhibiteur de B-Raf (BRAFi), un inhibiteur de MEK (MEKi) et un inhibiteur d'ERK (ERKi).

11. Procédé selon la revendication 10, dans lequel
ledit BRAFi est choisi dans le groupe comprenant le vémurafénib, le dabrafénib, l'encorafénib, le LGX818, le PLX4720, le TAK-632, le MLN2480, le SB590885, le XL281et le RAF265,
ledit MEKi est choisi dans le groupe comprenant l'AZD6244, le tramétinib, le sélumétinib, le cobimétinib, le binimétinib, le MEK162, le RO5126766, le GDC-0623, le PD 0325901, le CI-1040 et le TAK-733,
ledit ERKi est choisi dans le groupe comprenant l'ulixertinib, le SCH772984, le XMD8-92, le FR 180204, le GDC-0994, l'ERK5-IN-1, le DEL-22379, le BIX 02189, l'inhibiteur d'ERK (CAS n° 1049738-54-6), l'inhibiteur d'ERK III (CAS n° 331656-92-9), le GDC-0994 et le VTX11e, ou
ledit EGFRi est choisi dans le groupe comprenant le cétuximab, le panitumumab, le zalutumumab, le nimotuzumab, le matuzumab, le géfitinib, l'erlotinib, le lapatinib, l'AP26113, l'inhibiteur d'EGFR (CAS n° 879127-07-8), l'inhibiteur d'EGFR/ERbB-2, ErbB-4 (CAS n° 881001-19-0), l'inhibiteur d'EGFR/ErbB-2 (CAS n° 179248-61-4), l'inhibiteur d'EGFR II (BIBX 1382, CAS n° 196612-93-8), l'inhibiteur d'EGFR III (CAS n° 733009-42-2), l'inhibiteur d'EGFR/ErbB-2/ErbB-4 II (CAS n° 944341-54-2) et l'inhibiteur de PKCβII/EGFR (CAS n° 145915-60-2).
